# EUROPÄISCHE PATENTSCHRIFT

(11) **EP 0 710 087 B1**
(45) Veröffentlichungstag und Bekanntmachung des Hinweises auf die Patenterteilung: **05.02.1997**
(21) Anmeldenummer: 94919594.5
(22) Anmeldetag: 01.06.1994
(51) Int. Cl.: A61B 17/28

(54) **CHIRURGISCHES INSTRUMENT**
SURGICAL INSTRUMENT
INSTRUMENT CHIRURGICAL

(30) Priorität: 17.07.1993 DE 4324042
(43) Veröffentlichungstag der Anmeldung: 08.05.1996
(73) Patentinhaber: Schad, Gerhard, D-78600 Kolbingen (DE)
(72) Erfinder: Schad, Gerhard, D-78600 Kolbingen (DE)
(74) Vertreter: Böhme, Ulrich Hoeger, Stellrecht & Partner
(86) Internationale Anmeldenummer: EP9401777
(87) Internationale Veröffentlichungsnummer: WO9502365

(56) Entgegenhaltungen:
- EP-A- 0 541 930
- EP-A- 0 546 767
- EP-A- 0 609 503
- WO-A-91/02493
- DE-U- 9 114 306

## Beschreibung

Die Erfindung betrifft ein chirurgisches Instrument mit zumindestens einem über ein Zug- oder Druckelement betätigbaren Maulteil, wobei das Zug- oder Druckelement ein Rohr durchzieht, mit dem endwärtig das zumindest mit einem Maulteil lösbar verbunden ist und das von einem Außenrohr umfangen ist, welches verschiebbar auf dem Rohr sitzt.

Zangenähnliche chirurgische Instrumente sind in vielfältiger Form und Ausführung bekannt. Es handelt sich dabei um Greifinstrumente, Biopsieinstrumente, Schneidinstrumente, Nadel- und Fadenhalter, Faßzangen, Dissektionszangen, Löffelzangen usw. Alle diese Instrumente haben gemeinsam, daß mittels einer Betätigungseinrichtung Maulteile betätigt werden. Dabei kann es sich bei den Maulteilen um Scheren, Zangen, Schneiden, Klemmen od. dgl. handeln. In den meisten Fällen sind zwei Maulteile bewegbar, denkbar ist jedoch auch die Anordnung von nur einem Maulteil, welches gegenüber einem feststehenden Maulteil bewegbar ist.

Diese Maulteile werden in der Regel über ein Zug- oder Druckelement betätigt. Das Zugelement besteht dabei beispielsweise aus einem Drahtseil, ein Zug- oder Druckelement aus Drahtabschnitten, Stangen, Rohre od. dgl.

Betätigt werden diese Zug- oder Druckelemente und damit die entsprechenden Maulteile manuell über Scherengriffe oder elektrisch über Motoren, pneumatisch, hydraulisch od. dgl. Hier sind viele Ausgestaltungen möglich und sollen von der vorliegenden Erfindung umfaßt sein.

Alle diese genannten chirurgischen Instrumente müssen sowohl bei der Handhabung am oder im menschlichen Körper unterschiedlichste Aufgaben erfüllen, weshalb sie möglichst vielfältig anwendbar ausgestaltet sein sollen. In der heutigen Zeit wesentlich ist jedoch, daß trotz der vielfältigen Ausgestaltung das chirurgische Instrument leicht in Einzelteile zu zerlegen und zu reinigen ist. An die Hygiene und die Sterilisation werden heute sehr hohe Anforderungen gestellt, welche die gängigen chirurgischen Instrumente nicht erfüllen können. Entweder sind sie sehr kompliziert aufgebaut, können damit zwar unterschiedlichste Funktionen erfüllen, sind aber dementsprechend schwierig auseinanderzunehmen und wieder zusammenzusetzen. Sind sie dagegen einfach aufgebaut, können sie zwar auseinandergenommen werden, erfüllen aber in der Regel immer nur eine einzige Funktion und sind in der Handhabung erschwert.

In der EP 0 546 767 A2 sind chirurgische Instrumente dieser Art beschrieben, bei denen die eigentlichen zangen- oder scherenförmigen Werkzeuge drehbar an einer Halterung gelagert sind, diese ist in einem Rohrschaft des Instruments dauerhaft festgelegt, beispielsweise durch Einbörteln des Rohrschafts. Bei diesem Instrument sind die Halterungen mit den daran gelagerten Werkzeugen nicht auswechselbar, so daß beim Übergang zu einem anderen Instrument das ganze Instrument ausgewechselt werden muß. Ein Zerlegen zu Reinigungszwecken ist erschwert.

In der WO91/02493 sind derartige zangenförmige Instrumente beschrieben, bei denen die Werkzeuge lösbar an einem Rohrschaft gelagert sind. Allerdings ist das Einsetzen der Werkzeuge schwierig, da die Werkzeuge an einer Lagerwelle gehalten sind und mit einer Zahnung in Zahnstangen eingepaßt werden müssen, die am Instrument selbst verschiebbar gelagert sind. Es ist daher außerordentlich schwierig, diese Werkzeuge auszuwechseln.

Ausgehend von diesem Stand der Technik liegt der Erfindung die Aufgabe zugrunde, ein vielfältig anwendbares chirurgisches Instrument der gattungsgemäßen Art so auszubilden, daß es einerseits sehr gründlich zu reinigen beziehungsweise zu sterilisieren ist und andererseits das Auswechseln des Werkzeugs in einfacher Weise möglich wird.

Diese Aufgabe wird bei einem chirurgischen Instrument der eingangs beschriebenen Art gemäß einer ersten bevorzugten Lösungsform erfindungsgemäß dadurch gelöst, daß das Maulteil drehgelenkartig mit einer Halterung verbunden ist, daß die Halterung lösbar an das Rohr angekoppelt ist und daß die Halterung einen Wulstkeder aufweist, der in Gebrauchslage in einer Höhlung des Rohrs sitzt und von Schnappschenkeln des Rohrs gehalten ist.

Diese Aufgabe wird bei einem chirurgischen Instrument der eingangs beschriebenen Art gemäß einer zweiten bevorzugten Lösungsform erfindungsgemäß dadurch gelöst, daß das Maulteil drehgelenkartig mit einer Halterung verbunden ist, daß die Halterung lösbar an das Rohr angekoppelt ist und daß das Rohr endwärtig spannzangenartig ausgebildet ist, wobei die Spannzangen eine Anformung an der Halterung umgreifen.

Durch diese Ausgestaltung des chirurgischen Instrumentes ist vor allem ein Auswechseln einer Vielzahl von verschiedenen Maulteilen möglich, so daß mit dem Grundinstrument eine Vielzahl von chirurgischen Eingriffen durchgeführt werden kann. Ferner können hier auf leichte Art und Weise Elemente voneinander getrennt und gereinigt werden. Dies gilt vor allem für die Rohrseele und das Zugelement.

Wesentlich ist dabei, daß die Halterung für alle Maulteile gleich ausgebildet ist. Entsprechend der Funktion der Maulteile sind diese auch mit der Halterung über ein Drehgelenk, verbunden und an das Zugelement angekoppelt. Hier ist bereits eine Vielzahl von Betätigungsmöglichkeiten von Maulteilen bekannt und soll von der vorliegenden Erfindung umfaßt sein.

Das Zugelement und die Halterung können zusammen mit den Maulteilen von dem Rohr getrennt werden. Das Rohr und die Einheit aus Zugelement, Halterung und Maulteil werden dann getrennt gereinigt bzw. sterilisiert. Auf Wunsch kann nun mit einem Grundinstrument eine Reihe von verschiedenen Maulteilen mit Halterung und Zug- oder Druckelement angeboten werden. Je nach Wunsch erfolgt ein Austausch dieser unterschiedlichen Maulteile.

Zur Festlegung der Halterung an dem Rohr ist ein Verschluß vorgesehen, der aus einem Außenrohr besteht, welches das Innenrohr umgibt. Wird das Außenrohr verschoben, so übergreift es auch die Höhlung sowie den in der Höhlung sitzenden Wulstkeder der Halterung und die Schnappschenkel beziehungsweise die Spannzangen, so daß diese nicht aufgeweitet werden können. Auf diese Weise ist die Halterung an dem Innenrohr festgelegt.

In einem bevorzugten Ausführungsbeispiel sitzt das Innenrohr in einem Drehteil und ist über dieses Drehteil mit übrigen Teilen eines chirurgischen Instrumentes lösbar verbunden, wobei nicht nur das Drehteil von dem übrigen Teil gelöst werden kann, sondern auch das Zugelement. Dieses Drehteil kann, muß aber nicht, auch dem Drehen von dem Maulteil dienen. Es kann auch in einer bestimmten Stellung fest mit den übrigen Instrumententeilen verbunden sein, so daß auch diese Ausführungsform von der vorliegenden Erfindung umfaßt sein soll.

Bevorzugt weist das Drehteil eine das Innenrohr umgebende Ausnehmung auf, in welcher auch ein Endstück des Außenrohres angeordnet ist und sich über eine Feder, vorzugsweise eine Schraubenfeder, abstützt. Die Festlegung des Endstückes in der Ausnehmung erfolgt bajonettverschlußartig. Hierzu sind Hakenvorsprünge an dem Endstück in einer Ringnut in der Ausnehmung geführt. Beim Drehen des Endstückes können diese Hakenvorsprünge auf eine erste Randkante gebracht werden, bei der das Außenrohr die Höhlung und den Wulstkeder sowie die Schnappschenkel übergreift. Wird das Endstück weitergedreht, so gelangen die Hakenvorsprünge in den Bereich von Längsschlitzen, so daß das Außenrohr von dem Drehteil getrennt werden kann. Auf diese Art und Weise erfolgt eine getrennte Reinigung von Außenrohr und Innenrohr. Ferner ermöglicht diese Ausgestaltung auch eine Anordnung von unterschiedlichsten Dichtungen, Dichtringen usw., die verhindern, daß Verunreinigungen durch das Drehteil, den Zwischenraum zwischen Außenrohr und Innenrohr sowie das Innenrohr nach hinten in die übrigen Teile des chirurgischen Instrumentes gelangen.

Im bevorzugten Ausführungsbeispiel ist das Drehteil, wie der Name schon sagt, drehbar angeordnet, so daß die Maulteile eine unterschiedliche Stellung einnehmen können. Die Drehung kann um 360 Grad erfolgen. Eine Drehbegrenzung geschieht über Rastnasen, welche in Rastmulden eines Schubteiles eingreifen. Dieses Schubteil sitzt wiederum lösbar einem Klemmteil auf, welches der lösbaren Halterung des Drehteiles dient. Hierzu übergreift das Schubteil einen Rohrabschnitt des Klemmteils, in den das Drehteil mit einem Bolzenabschnitt eingesetzt ist. Dieser Bolzenabschnitt weist einen Ringkanal auf, in den mittels des Schubteiles Kugeln eingedrückt werden. Dies geschieht über eine Konusbohrung in dem Schubteil. Die Kugeln sitzen in entsprechenden Kugelaufnahmen in dem Rohrabschnitt.

Das Schubteil ist im übrigen mit dem Klemmteil lösbar verbunden. Dies geschieht über einen Sprengring, der in eine Ringnut des Rohrabschnittes eingesetzt werden kann und in Gebrauchslage einen Anschlagring innerhalb des Schubteiles hintergreift. Ferner stützt sich das Schubteil über eine Feder, bevorzugt eine Schraubenfeder, gegen das Klemmteil ab und wird über diese Schraubenfeder gegen das Drehteil gedrückt, so daß die Rastmulden die oben erwähnten Rastnasen aufnehmen.

In dem Klemmteil lagert wiederum verschiebbar ein Einsatz, welcher das Zug- bzw. Druckelement mit einer Betätigungseinrichtung verbindet. Hierzu besteht der Einsatz aus einem Spannzangenteil mit einer Höhlung zur Aufnahme einer dem Zug- bzw. Druckelement angeformten Kugel. Die Zangenschenkel des Spannzangenteiles bzw. ein entsprechender Spannzangenkopf steht unter dem Druck von Führungsnocken, die vom Schubteil durch Längsschlitze in dem Rohrabschnitt des Klemmteils auf die Zangenschenkel einwirken. Wird das Schubteil verschoben, so läßt der Druck der Führungsnocken auf den Spannzangenkopf nach, da auf diesen Spannzangenkopf eine Ringmulde folgt. Durch einfachen Zug gleitet die Kugel aus der Kugelhöhlung, so daß das Zugelement von dem Einsatz gelöst ist.

In dem Einsatz lagert verschiebbar eine Verbindungsstange zu einer Betätigungseinrichtung. Durch ein Verschieben dieser Verbindungsstange und ein danach erfolgtes Festlegen kann die Öffnungsweite der Maulteile bestimmt werden.

Im vorliegenden Ausführungsbeispiel schließt dann an die Verbindungsstange eine Kugel an, welche mit einem Griffteil lösbar verbunden ist. In diesem Ausführungsbeispiel besteht die Betätigungseinrichtung der Einfachheit halber aus zwei Griffteilen, jedoch sind auch alle übrigen oben erwähnten Betätigungseinrichtungen denkbar.

Die Betätigung des Einsatzes erfolgt wiederum gegen den Druck einer Feder, die sich zwischen dem Spannzangenteil und einem Deckel abstützt, wobei der Deckel das Klemmteil verschließt und von der Verbindungsstange durchsetzt ist.

Insgesamt ist durch diese Ausgestaltung ein chirurgisches Instrument geschaffen, welches außerordentlich viele Anwendungsmöglichkeiten bietet, sehr flexibel zu handhaben ist, außerordentlich leicht auseinanderzunehmen und zu reinigen ist. Auch sein Zusammenbau bietet keinerlei Schwierigkeiten, wobei vor allem die Auswechselbarkeit der Maulteile hervorzuheben ist.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
- Fig. 1:: eine Draufsicht auf ein erfindungsgemäßes chirurgisches Instrument;
- Fig. 2:: einen Längsschnitt durch Teile des chirurgischen Instrumentes gemäß Figur 1, teilweise in Explosionsdarstellung;
- Fig. 3:: eine Draufsicht auf ein Drehteil für das chirurgische Instrument gemäß Figur. 1;
- Fig. 4:: einen Querschnitt durch das Drehteil gemäß Figur 3 entlang Linie IV - IV;
- Fig. 5:: eine Draufsicht auf ein Schubteil für das chirurgische Instrument gemäß Figur 1;
- Fig. 6:: eine Stirnansicht des Schubteils gemäß Figur 5 entlang Linie VI - VI;
- Fig. 7:: eine Draufsicht auf ein Klemmteil für das chirurgische Instrument gemäß Figur 1;
- Fig. 8:: einen Querschnitt durch das Klemmteil gemäß Figur 7 entlang Linie VIII - VIII;
- Fig. 9:: eine Draufsicht auf einen Einsatz in das Klemmteil gemäß Figur 7;
- Fig. 10:: eine Draufsicht auf eine Funktionsdarstellung des chirurgischen Instrumentes gemäß Figur 1.

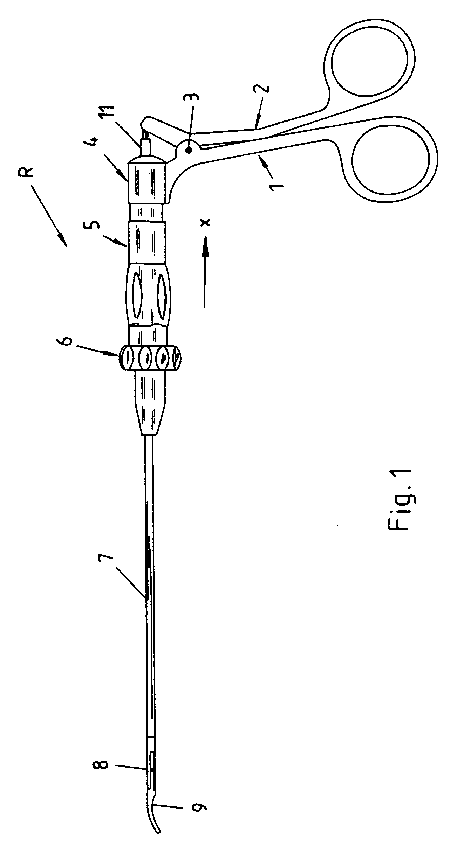

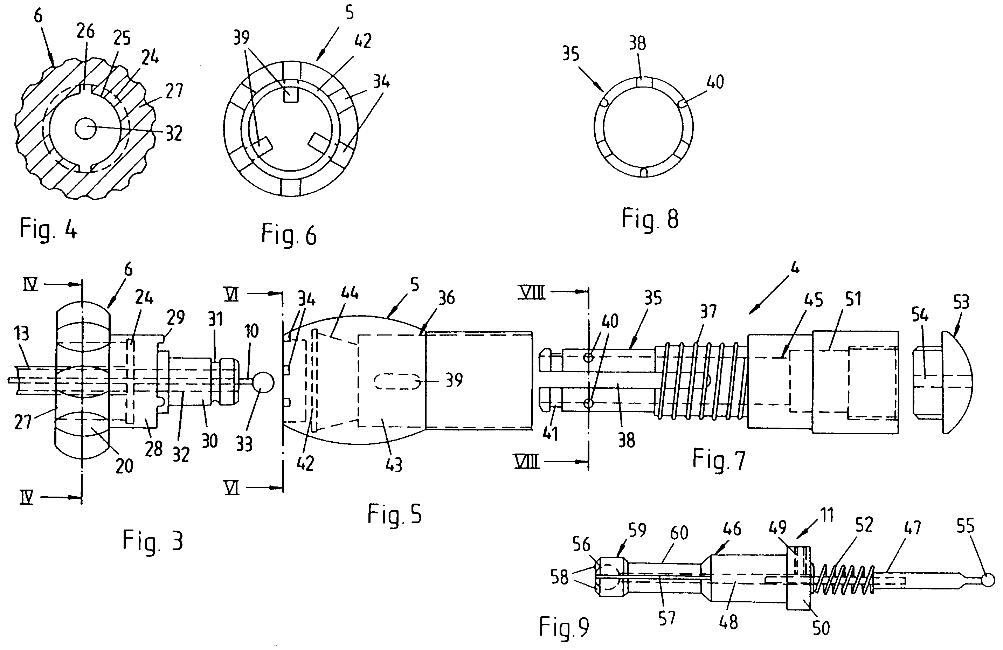

Ein chirurgisches Instrument R weist gemäß Figur 1 zwei Griffteile 1 und 2 auf, welche über ein Drehgelenk 3 miteinander verbunden sind. Das Griffteil 1 ist fest mit einem Klemmteil 4 verbunden, auf dem ein in Richtung x verschiebbares Schubteil 5 teilweise aufsitzt. An das Schubteil 5 schließt ein Drehteil 6 an. Von diesem führt ein Außenrohr 7 zu einer Halterung 8 für Maulteile 9, welche beliebig ausgestaltet sein können. Die Maulteile 9 stehen über ein in Fig. 3 gezeigtes Zugelement 10 mit einem Einsatz 11 in Verbindung, der wiederum gelenkig mit dem Griffteil 2 verbunden ist.

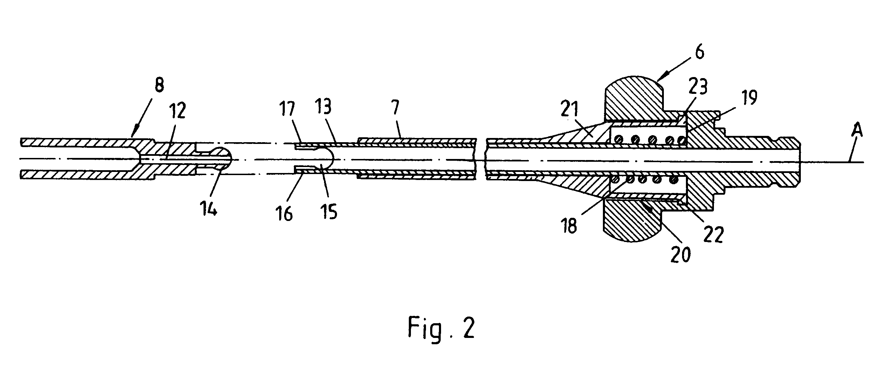

In Figur 2 ist erkennbar, daß die Halterung 8 gabelförmig ausgebildet ist. Dies ist jedoch nur ein Ausführungsbeispiel, von der vorliegenden Erfindung sollen alle möglichen Halterungen, alle möglichen Maulteile und alle möglichen drehgelenkartigen Verbindungen zwischen den Maulteilen und der Halterung bzw. dem Zugelement 10 umfaßt sein. Hierzu zählen beispielsweise bekannte Faßzangen, Zangen zur Probeexcision, Biopsiezangen, Gewebefaßzangen, Nadelhalter, Scheren, Dissektionszangen usw. Nur beispielhaft wird auf eine Biopsiezange gemäß der europäischen Patentanmeldung 0 065 054 bzw. FR-PS 2 479 608 oder auf ein chirurgisches Instrument gemäß der europäischen Patentanmeldung 0 119 405 verwiesen. All diese chirurgischen Instrumente arbeiten mit Maulteilen, welche eine schneidende, klemmende, scherende oder dergleichen Funktion ausüben und von einem Zugelement betätigt werden.

Im vorliegenden Ausführungsbeispiel ist das Zugelement 10 als ein Draht ausgebildet, es könnte jedoch auch ein Seil oder ein Rohr sein. Zur Anlenkung an die in Figur 2 nicht näher gezeigten Maulteile durchfährt das Zugelement 10 eine Innenbohrung 12 innerhalb der Halterung 8.

Ein wesentliches Merkmal der vorliegenden Erfindung ist eine lösbare Ankopplung der Halterung 8 an die übrigen Teile des chirurgischen Instrumentes R. Im vorliegenden Ausführungsbeispiel weist die Halterung zur lösbaren Ankopplung an ein Innenrohr 13 einen Wulstkeder 14 auf, der ebenfalls von der Innenbohrung 12 durchsetzt ist. Dieser Wulstkeder 14 kann in eine entsprechende Höhlung 15 in das Innenrohr 13 eingeschoben werden, wobei zwei Schnappschenkel 16 und 17 den Wulstkeder 14 zum Festlegen übergreifen.

Die Schnappschenkel 16 und 17 sind federnd ausgebildet, so daß die Halterung 8 beim Ausfedern der Schnappschenkel 16 und 17 aus der Höhlung 15 herausgezogen werden kann.

Eine Festlegung des Wulstkeders 14 in der Höhlung 15 erfolgt durch das Außenrohr 7. Dieses Außenrohr 7 sitzt, wie das Innenrohr 13 auch, in dem Drehteil 6. Während jedoch das Innenrohr 13 im Drehteil 6 festliegt, stützt sich das Außenrohr 7 über eine Schraubenfeder 18, welche das Innenrohr 13 umgibt, gegen einen Boden 19 in einer Ausnehmung 20 in dem Drehteil 6 ab.

In der in Fig. 2 gezeigten Gebrauchslage, in welcher die Höhlung 15 freigegeben ist und in welcher die Halterung 8 von dem Innenrohr 13 getrennt werden kann, befindet sich ein Endstück 21 des Außenrohres 7 gegen den Druck der Schraubenfeder 18 gänzlich in der Ausnehmung 20 und hintergreift dort mit Hakenvorsprüngen 22 hinterschnittene Randkanten 23.

Durch Drehen des Endstückes 21 werden auch die Hakenvorsprünge 22 in einer entsprechenden, in Fig. 3 und 4 gezeigten Ringnut 24 gedreht und gelangen zu einer zweiten hinterschnittenen Randkante 25, welche weiter vom Boden 19 entfernt ist. Die Schraubenfeder 18 drückt dabei die Hakenvorsprünge 22 gegen diese Randkante 25, wobei das Außenrohr 7 entgegen der Schubrichtung x zur Halterung 8 hin verschoben ist und dabei die Höhlung 15 und die Schnappschenkel 16 und 17 übergreift. Dieses Übergreifen verhindert ein Ausfedern der Schnappschenkel 16 und 17, so daß der Wulstkeder fest in der Höhlung 15 gehalten ist.

Das Endstück 21 kann noch eine weitere Drehung vollführen, so daß die Hakenvorsprünge 22 in Bereiche von Längsschlitzen 26 (siehe Figur 4) gelangen, wobei das Endstück 21 und damit das Außenrohr 7 von dem Drehteil 6 gelöst werden kann. Hierdurch kann eine bessere Reinigung der Seele des Außenrohres 7 und der Ausnehmung 20 erfolgen.

Ein weiteres Merkmal der vorliegenden Erfindung liegt darin, daß das Innenrohr 13 und damit auch die Halterung 8 bzw. die Maulteile 9 mittels des Drehteils 6 um eine Längsachse A drehbar angeordnet sind. Hierzu ist, wie oben erwähnt, das Innenrohr 13 in dem Drehteil 6 festgelegt. Das Drehteil 6 weist einen Drehring 27 mit entsprechenden Riffelungen auf, an denen ein Ringwall 28 mit Rastnasen 29 anschließt. Auf den Ringwall 28 folgt mit einem geringeren Durchmesser ein Bolzenabschnitt 30 mit einem Ringkanal 31.

Die Ausnehmung 20 greift zusammen mit der Ringnut 24 zumindest teilweise in den Ringwall 28 an, daran schließt sich eine Axialbohrung 32 in dem Ringwall 28 und dem Bolzenabschnitt 30 an, welche von dem Zugelement 10 durchzogen ist. Dem Zugelement 10 ist dann endwärtig eine Kugel 33 aufgesetzt.

In Gebrauchslage greift der Bolzenabschnitt 30 in das Schubteil 5 gem. Fig. 5 ein, wobei die Rastnasen 29 in Rastmulden 34 aufgenommen werden. Wird das Schubteil 5 ein wenig in Richtung x verschoben, werden die Rastnasen 29 freigegeben, und das Drehteil 6 kann um die Längsachse A gedreht werden.

Das Schubteil 5 ist dem Klemmteil 4 gemäß Figur 7 aufgeschoben, wobei dieses Klemmteil 4 mit einem Rohrabschnitt 35 in eine Stufenbohrung 36 des Schubteils 5 eingreift. Hierbei stützt sich das Schubteil 5 gegenüber dem Klemmteil 4 über eine Schraubenfeder 37 ab, welche den Rohrabschnitt 35 umfängt. Die Schraubenfeder 37 drückt das Schubteil 5 entgegen der Schubrichtung x.

Im vorliegenden Ausführungsbeispiel sind in den Rohrabschnitt 35 drei Längsschlitze 38 eingeformt, in welche in Gebrauchslage Führungsnocken 39 eingreifen, die in das Schubteil 5 eingesetzt sind. Diese Führungsnocken 39 weisen noch eine weitere, den Einsatz 11 betreffende Funktion auf, die später beschrieben wird.

Ferner sind in den Rohrabschnitt 35 Kugelaufnahmen 40 und eine Ringnut 41 eingeformt. Die Ringnut 41 dient der Aufnahme eines nicht näher gezeigten Sprengringes, der in die Ringnut 41 nach dem Einsetzen des Klemmteils 4 eingesetzt wird. Hierbei hintergreift der Sprengring einen Anschlagring 42 (siehe Fig. 6), der in die lichte Weite der Stufenbohrung 36 des Schubteils 5 einragt. Der Sprengring verhindert damit, daß das Schubteil 5 von dem Klemmteil 4 gelöst wird.

Zwischen einer Aufnahmekammer 43 der Stufenbohrung 36 für die Schraubenfeder 37 und dem Anschlagring 42 ist die Stufenbohrung 36 als Konusbohrung 44 ausgestaltet. Diese Konusbohrung 44 wirkt mit nicht näher gezeigten Kugeln in den Kugelaufnahmen 40 zusammen. Wird das Schubteil 5 entgegen der Schubrichtung x verschoben, so drückt die Konusbohrung 44 die Kugeln in die Kugelaufnahmen 40, wobei die Kugeln andererseits in den Ringkanal 31 des Bolzenabschnittes 30 des Drehteils 6 eingedrückt werden. Hierdurch wird das Drehteil 6 lösbar mit dem Klemmteil 4 verbunden.

Auch das Klemmteil 4 ist von einer Stufenbohrung 45 durchzogen. In dieser Stufenbohrung 45 sitzt der Einsatz 11 gemäß. Fig. 9. Dieser Einsatz 11 besteht aus einem Spannzangenteil 46 und einer Verbindungsstange 47, welche verschiebbar in einer Längsbohrung 48 des Spannzangenteils 46 sitzt. Die Festlegung der Verbindungsstange 47 in der Längsbohrung 48 erfolgt durch eine Madenschraube 49. Die Madenschraube 49 durchsetzt einen Flansch 50, der in Gebrauchslage in einer Innenkammer 51 der Stufenbohrung 45 des Klemmteils 4 gleiten kann. Das Gleiten geschieht gegen den Druck einer Schraubenfeder 52, die sich einerseits gegen den Flansch 50 und andererseits gegen einen in die Stufenbohrung 45 eingeschraubten Deckel 53 abstützt. Dabei durchsetzt die Verbindungsstange 47 eine Bohrung 54 in dem Deckel 53 und sitzt schlußendlich in Gebrauchslage mit einer Kugel 55 in einer Kugelaufnahme des Griffteiles 2.

Das Spannzangenteil 46 dient der Festlegung des Zugelementes 10, wobei die Kugel 33 in einer Kugelhöhlung 56 aufgenommen wird. Zu diesem Zweck sind im Spannzangenteil 46 durch Schlitzungen 57 Zangenschenkel 58 ausgebildet, welche sich federnd aufspreizen können. Ein derartiges Aufspreizen wird jedoch durch die Führungsnocken 39 so lange verhindert, solange das Schubteil 5 an dem Ringwall 28 des Drehteiles 6 anschlägt. In dieser Gebrauchslage drücken die Führungsnocken 39 auf einen Spannzangenkopf 59, so daß die Zangenschenkel 58 in Schließlage verbleiben.

Wird jedoch das Schubteil 5 in Schubrichtung x auf dem Klemmteil 4 verschoben, so gleiten die Führungsnocken 39 in eine Ringmulde 60 des Spannzangenteiles 46 ein und geben die Zangenschenkel 58 in begrenztem Umfange frei. Diese können dann auffedern, so daß die Kugel 33 aus der Kugelhöhlung 56 herausgleiten kann.

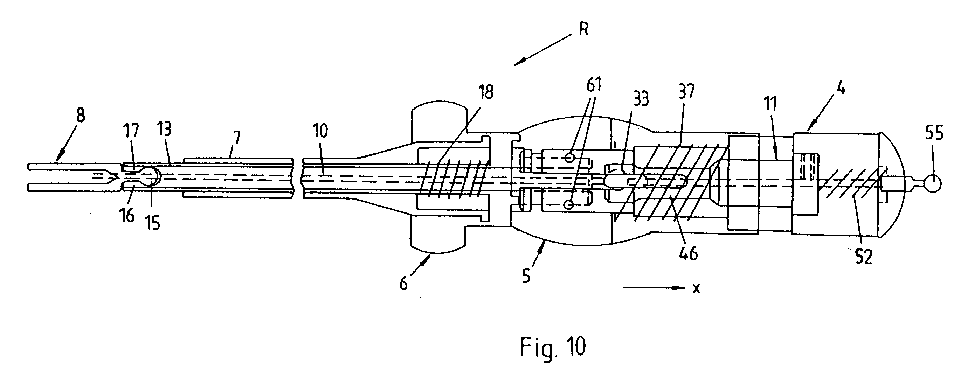

In Fig. 10 ist das Funktionsprinzip der vorliegenden Erfindung schematisch angedeutet. Über die Kugel 55 des Einsatzes 11 sind das Zugelement 10 und damit die nicht näher gezeigten Maulteile 9 mit dem ebenfalls nicht näher gezeigten Griffteil 2 verbunden, welches über das Drehgelenk 3 mit dem anderen Griffteil 1 eine Gelenkverbindung eingeht. Durch Betätigen der Griffteile 1 und 2 wird der Einsatz 11 gegen den Druck der Schraubenfeder 52 bewegt, wobei er das Zugelement 10 mit sich nimmt und hierdurch die Maulteile 9 betätigt. Da das Zugelement 10 mit seiner Kugel 33 beweglich von dem Spannzangenteil 46 gehalten wird, kann das Zugelement 10 ohne weiteres um die Längsachse A drehen, so daß die Stellung der Maulteile 9 veränderlich ist. Dies geschieht durch Drehen des Drehteiles 6, wobei das Schubteil 5 geringfügig in Richtung x verschoben ist, so daß die Rastnasen 29 frei sind. Wird dann das Schubteil 5 losgelassen, so gelangt es unter dem Druck der Schraubenfeder 37 wieder in die Ausgangslage zurück, wobei die Rastnasen 29 in die Rastmulden 34 einfahren.

Zum Auswechseln der Maulteile 9 bzw. zum Reinigen des gesamten chirurgischen Instrumentes R wird das Schubteil 5 ganz in Richtung x gegen den Druck der Schraubenfeder 37 verschoben. Hierbei werden sowohl Kugeln 61 in den Kugelaufnahmen 40 freigegeben, die damit aus dem Ringkanal 31 des Drehteiles 6 herausgleiten können. Gleichzeitig gelangen aber auch die Führungsnocken 39 in den Bereich der Ringmulde 60, so daß die Zangenschenkel 58 frei sind und sich aufspreizen können. Hierdurch kann die Kugel 33 aus der entsprechenden Kugelhöhlung 56 gezogen werden. Damit ist der Bereich des Drehteiles 6 mit Außenrohr und Innenrohr sowie Zugelement 10 von dem Bereich mit Schubteil 5, Klemmteil 4 und Einsatz 11 getrennt.

Soll nun das Zugelement 10 zusammen mit der Halterung 8 von dem Innenrohr 13 getrennt werden, so wird das Außenrohr 7 gegen den Druck der Schraubenfeder 18 in das Drehteil 6 eingedrückt, so daß die Höhlung 15 mit dem Wulstkeder 14 und die Schnappschenkel 16 und 17 freigegeben sind. Jetzt kann die Halterung 8 zusammen mit dem Zugelement 10 von dem Innenrohr 13 getrennt und durch andere Maulteile mit einer entsprechenden Halterung 8 und einem Zugelement 10 ersetzt werden.

Zum Reinigen wird das Außenrohr 7 noch weitergedreht, so daß die Hakenvorsprünge 22 durch die Längsschlitze 26 gleiten können, wodurch das Außenrohr 7 von dem Drehteil 6 getrennt wird.

Es versteht sich von selbst, daß insbesondere im Drehteil entsprechende Dichtringe und Dichtungsanordnungen sowohl zwischen Innenrohr 13 als auch Außenrohr 7 als auch zwischen Innenrohr 13 und Zugelement 10 angeordnet sind, welche vermeiden, daß Blut, Gewebeflüssigkeit od. dgl. durch das Innenrohr 13 bzw. zwischen Innenrohr 13 und Außenrohr 7 oder auch durch das Drehteil 6 nach hinten in den Bereich des Schubteiles 5 gelangen kann.

## Patentansprüche

1. Chirurgisches Instrument mit zumindest einem über ein Zug- oder Druckelement (10) betätigbaren Maulteil (9), wobei das Zug- oder Druckelement (10) ein Rohr (13) durchzieht, mit dem endwärtig das zumindest eine Maulteil (9) lösbar verbunden ist und das von einem Außenrohr (7) umfangen ist, welches verschiebbar auf dem Rohr (13) sitzt, dadurch gekennzeichnet, daß das Maulteil (9) drehgelenkartig mit einer Halterung (8) verbunden ist, daß die Halterung (8) lösbar an das Rohr (13) angekoppelt ist und daß die Halterung (8) einen Wulstkeder (14) aufweist, der in Gebrauchslage in einer Höhlung (15) des Rohrs (13) sitzt und von Schnappschenkeln (16, 17) des Rohrs (13) gehalten ist.

2. Chirurgisches Instrument mit zumindest einem über ein Zug- oder Druckelement (10) betätigbaren Maulteil (9), wobei das Zug- oder Druckelement (10) ein Rohr (13) durchzieht, mit dem endwärtig das zumindest eine Maulteil (9) lösbar verbunden ist und das von einem Außenrohr (7) umfangen ist, welches verschiebbar auf dem Rohr (13) sitzt, dadurch gekennzeichnet, daß das Maulteil (9) drehgelenkartig mit einer Halterung (8) verbunden ist, daß die Halterung (8) lösbar an das Rohr (13) angekoppelt ist und daß das Rohr (13) endwärtig spannzangenartig ausgebildet ist, wobei die Spannzangen eine Anformung an der Halterung (8) umgreifen.

3. Instrument nach Anspruch 1 oder 2, dadurch gekennzeichnet, daß das Zug- oder Druckelement (10) eine Innenbohrung (12) in der Halterung (8) durchzieht.

4. Instrument nach wenigstens einem der voranstehenden Ansprüche, dadurch gekennzeichnet, daß das Rohr (13) mit einem Drehteil (6) drehfest verbunden ist.

5. Instrument nach Anspruch 4, dadurch gekennzeichnet, daß das Drehteil (6) eine das Rohr (13) umgebende Ausnehmung (20) aufweist, in welcher sich das Außenrohr (7) gegen eine Feder (18) abstützt und lösbar geführt ist.

6. Instrument nach Anspruch 5, dadurch gekennzeichnet, daß das Außenrohr (7) mit Hakenvorsprüngen (22) in einer Ringnut (24) in der Ausnehmung (20) geführt ist, wobei die Ringnut (24) hinterschnittene Randkanten (23, 25) aufweist und in die Ausnehmung (20) achsparallele Längsschlitze (26) eingeformt sind.

7. Instrument nach Anspruch 5 oder 6, dadurch gekennzeichnet, daß das Drehteil (6) lösbar mit einem Klemmteil (4) verbunden ist.

8. Instrument nach Anspruch 7, dadurch gekennzeichnet, daß das Schubteil (5) Rastmulden (34) zur Aufnahme von Rastnasen (29) an dem Drehteil (6) aufweist.

9. Instrument nach Anspruch 7 oder 8, dadurch gekennzeichnet, daß einem Rohrabschnitt (35) des Klemmteils (4) ein Schubteil (5) aufgesetzt ist und sich über eine Feder (37) gegen das Klemmteil (4) abstützt.

10. Instrument nach Anspruch 9, dadurch gekennzeichnet, daß das Schubteil (5) mittels einer Konusbohrung (44) Kugeln (61) in Kugelaufnahmen (40) in dem Rohrabschnitt (35) beaufschlagt und diese in einen Ringkanal (31) in einem Bolzenabschnitt (30) des Drehteiles (6) eindrückt.

11. Instrument nach Anspruch 9 oder 10, dadurch gekennzeichnet, daß der Rohrabschnitt (35) eine Ringnut (41) zum Einsetzen eines Sprengringes aufweist, welcher in Gebrauchslage einen Anschlagring (42) in dem Schubteil (5) hintergreift.

12. Instrument nach wenigstens einem der Ansprüche 7 bis 11, dadurch gekennzeichnet, daß in dem Klemmteil (4) ein Einsatz (11) verschiebbar angeordnet ist, welcher das Zug- oder Druckelement (10) mit einer Betätigungseinrichtung (1, 2) lösbar verbindet.

13. Instrument nach Anspruch 12, dadurch gekennzeichnet, daß der Einsatz (11) ein Spannzangenteil (46) aufweist, welches eine Kugelhöhlung (56) zur Aufnahme einer dem Zug- oder Druckelement (10) angeformten Kugel (33) besitzt.

14. Instrument nach Anspruch 13, dadurch gekennzeichnet, daß die Zangenschenkel (58) des Spannzangenteiles (46) unter dem Druck von Führungsnocken (39) des Schubteiles (5) stehen.

15. Instrument nach Anspruch 14, dadurch gekennzeichnet, daß die Führungsnocken (39) Längsschlitze (38) in dem Rohrabschnitt (35) des Klemmteiles (4) durchgreifen und einen Spannzangenkopf (59) mit Druck beaufschlagen, wobei an dem Spannzangenkopf (59) eine Ringmulde (60) anschließt.

16. Instrument nach einem der Ansprüche 13 bis 15, dadurch gekennzeichnet, daß mit dem Spannzangenteil (46) eine Verbindungsstange (47) lösbar verbunden ist, die eine Kugel (55) zur Verbindung mit einen Griffteil (2) aufweist.

17. Instrument nach Anspruch 16, dadurch gekennzeichnet, daß sich das Spannzangenteil (46) über eine Feder (52) gegen einen Deckel (53) abstützt, der von der Verbindungsstange (47) durchsetzt ist.

## Claims

1. A surgical instrument comprising at least one jaw part (9) operable by a pushing or pulling member (10), wherein the pushing or pulling member (10) extends through an inner tube (13), to the end of which the at least one jaw part (9) is detachably connected and which is surrounded by an outer tube (7) displaceably mounted on the inner tube (13), characterised in that the jaw part (9) is pivotably connected to a mounting (8), in that the mounting (8) is detachably connected to the inner tube (13) and in that the mounting (8) has a bulbous tip (14) which, in the position of use, is arranged in a cavity (15) in the inner tube (13) and is retained by snap arms (16, 17) of the inner tube (13).

2. A surgical instrument comprising at least one jaw part (9) operable by a pushing or pulling member (10), wherein the pushing or pulling member (10) extends through an inner tube (13), to the end of which the at least one jaw part (9) is detachably connected and which is surrounded by an outer tube (7) displaceably mounted on the inner tube (13), characterised in that the jaw part (9) is pivotably connected to a mounting (8), in that the mounting (8) is detachably connected to the inner tube (13) and in that the end of the inner tube (13) is formed in the manner of a spring chuck engaging round a moulding on the mounting (8).

3. An instrument according to claim 1 or 2, characterised in that the pushing or pulling member (10) extends through an inner bore (12) in the mounting (8).

4. An instrument according to at least one of the preceding claims, characterised in that the inner tube (13) is connected in a rotationally fixed manner to a rotary part (6).

5. An instrument according to claim 4, characterised in that the rotary part (6) has a recess (20) which surrounds the inner tube (13) and in which the outer tube (7) is supported against a spring (18) and is detachably guided.

6. An instrument according to claim 5, characterised in that the outer tube (7) is guided in an annular groove (24) in the recess (20) by means of hook-type projections (22), the annular groove (24) having undercut lateral edges (23, 25), and axially parallel longitudinal slots (26) being formed in the recess (20).

7. An instrument according to claim 5 or 6, characterised in that the rotary part (6) is detachably connected to a clamping part (4).

8. An instrument according to claim 7, characterised in that [a] sliding part (5) has locking recesses (34) for receiving locking projections (29) on the rotary part (6).

9. An instrument according to claim 7 or 8, characterised in that a sliding part (5) is mounted on a tubular portion (35) of the clamping part (4) and is supported thereagainst by a spring (37).

10. An instrument according to claim 9, characterised in that the sliding part (5) acts upon balls (61) in ball mounts (40) in the tubular portion (35) by means of a conical bore (44) and pushes them into an annular channel (31) in a bolt portion (30) of the rotary part (6).

11. An instrument according to claim 9 or 10, characterised in that the tubular portion (35) has an annular groove (41) for receiving a snap ring engaging behind a stop ring (42) in the sliding part (5) in the position of use.

12. An instrument according to at least one of claims 7 to 11, characterised in that an insert (11) is displaceably arranged in the clamping part (4) and detachably connects the pushing or pulling member (10) to an operating device (1, 2).

13. An instrument according to claim 12, characterised in that the insert (11) has a spring-chuck part (46) provided with a spherical cavity (56) for receiving a ball (33) formed on the pushing or pulling member (10).

14. An instrument according to claim 13, characterised in that the chuck arms (58) of the spring-chuck part (46) are loaded by guide pins (39) of the sliding part (5).

15. An instrument according to claim 14, characterised in that the guide pins (39) extend through longitudinal slots (38) in the tubular portion (35) of the clamping part (4) and load a spring-chuck head (59), an annular trough (60) being adjacent thereto.

16. An instrument according to any one of claims 13 to 15, characterised in that a connecting rod (47) is detachably connected to the spring-chuck part (46) and has a ball (55) for connection to a handle part (2).

17. An instrument according to claim 16, characterised in that the spring-chuck part (46) is supported by a spring (52) against a cover (53), through which the connecting rod (47) extends.

## Revendications

1. Instrument chirurgical comprenant au moins une partie de travail ayant la forme d'un bec, d'une mâchoire, d'une lame ou analogue et appelée bec (9), qui est manoeuvrable par l'intermédiaire d'un élément de traction ou de poussée (10) traversant un tube (13) à l'extrémité duquel le bec (9) est relié de façon détachable et qui est entouré d'un tube extérieur (7) disposé coulissant sur ce tube (13), caractérisé en ce que le bec (9) est relié à un support (8) par un moyen semblable à une articulation, que le support (8) est accouplé amovible au tube (13) et que le support (8) présente un appendice pourvu d'un renflement (14) qui, à la position d'utilisation, est situé dans une cavité (15) du tube (13) et maintenu par des branches à déclic (16, 17) de ce tube (13).

2. Instrument chirurgical comprenant au moins une partie de travail ayant la forme d'un bec, d'une mâchoire, d'une lame ou analogue et appelée bec (9), qui est manoeuvrable par l'intermédiaire d'un élément de traction ou de poussée (10) traversant un tube (13) à l'extrémité duquel le bec (9) est relié de façon détachable et qui est entouré d'un tube extérieur (7) disposé coulissant sur ce tube (13), caractérisé en ce que le bec (9) est relié à un support (8) par un moyen semblable à une articulation, que le support (8) est accouplé amovible au tube (13) et que le tube (13) est réalisé à son extrémité à la façon d'une pince de serrage entourant une protubérance formée sur le support (8).

3. Instrument selon la revendication 1 ou 2, caractérisé en ce que l'élément de traction ou de poussée (10) traverse un perçage interne (12) du support (8).

4. Instrument selon au moins une des revendications précédentes, caractérisé en ce que le tube (13) est solidarisé en rotation avec une pièce de rotation (6).

5. Instrument selon la revendication 4, caractérisé en ce que la pièce de rotation (6) présente un évidement (20) qui entoure le tube (13) et dans lequel le tube extérieur (7) est appuyé sur un ressort (18) et guidé de façon amovible.

6. Instrument selon la revendication 5, caractérisé en ce que le tube extérieur (7) est guidé par des saillies formant des crochets (22) dans une rainure annulaire (24) à l'intérieur de l'évidement (20), la rainure (24) présentant des bords rentrants (23, 25) et des fentes longitudinales (26) parallèles à l'axe étant formées dans l'évidement (20).

7. Instrument selon la revendication 5 ou 6, caractérisé en ce que la pièce de rotation (6) est reliée amovible à une pièce de serrage (4).

8. Instrument selon la revendication 7, caractérisé en ce qu'il comprend une pièce coulissante de translation (5) qui présente des encoches d'arrêt (34) destinées à recevoir des ergots d'arrêt (29) prévus sur la pièce de rotation (6).

9. Instrument selon la revendication 7 ou 8, caractérisé en ce qu'une pièce coulissante de translation (5) est placée sur une partie tubulaire (35) de la pièce de serrage (4), sur laquelle elle s'appuie par l'intermédiaire d'un ressort (37).

10. Instrument selon la revendication 9, caractérisé en ce que la pièce coulissante (5) agit par une surface intérieure conique (44) sur des billes (61) maintenues dans des logements de billes (40) ménagés dans la partie tubulaire (35) afin de les pousser dans un canal annulaire (31) d'une queue (30) de la pièce de rotation (6).

11. Instrument selon la revendication 9 ou 10, caractérisé en ce que la partie tubulaire (35) présente une gorge annulaire (41) pour la mise en place d'un segment d'arrêt qui, à la position d'utilisation, se trouve derrière un anneau de butée (42) situé à l'intérieur de la pièce coulissante (5).

12. Instrument selon au moins une des revendications 7 à 11, caractérisé en ce que la pièce de serrage (4) contient un insert coulissant (11) qui relie l'élément de traction ou de poussée (10) de façon détachable à un dispositif de manoeuvre (1, 2).

13. Instrument selon la revendication 12, caractérisé en ce que l'insert (11) comporte une partie (46) formant une pince de serrage et possédant une cavité sphérique (56) pour la réception d'une sphère (33) formée sur l'élément de traction ou de poussée (10).

14. Instrument selon la revendication 13, caractérisé en ce que les mâchoires (58) de la partie (46) formant pince de serrage, sont exposées à la pression de taquets de guidage (39) de la pièce coulissante (5).

15. Instrument selon la revendication 14, caractérisé en ce que les taquets de guidage (39) traversent des fentes longitudinales (38) de la partie tubulaire (35) de la pièce de serrage (4) et exercent une pression sur une tête de pince de serrage (59), la tête de pince (59) se raccordant à un creux annulaire (60).

16. Instrument selon une des revendications 13 à 15, caractérisé en ce qu'il comprend une tige de liaison (47) reliée de façon détachable à la partie (46) formant pince de serrage et présentant une sphère (55) pour l'attache à une pièce de préhension et de manoeuvre (2).

17. Instrument selon la revendication 16, caractérisé en ce que la partie (46) formant pince de serrage s'appuie par l'intermédiaire d'un ressort (52) sur un bouchon (53) traversé de la tige de liaison (47).
